Europäisches Patentamt

European Patent Office

Office européen des brevets

(19)

(11) Numéro de publication: **0 215 722**
A1

## (12) DEMANDE DE BREVET EUROPEEN

(21) Numéro de dépôt: 86450016.0

(22) Date de dépôt: 30.07.86

(51) Int. Cl.⁴: **C 07 H 3/02**, C 07 D 307/32

(30) Priorité: **20.09.85 FR 8514098**

(43) Date de publication de la demande: **25.03.87**
**Bulletin 87/13**

(84) Etats contractants désignés: **BE CH DE GB IT LI LU NL**

(71) Demandeur: **SOCIETE CORTIAL S.A., 7 rue de l'Armorique, F-75015 Paris (FR)**

(72) Inventeur: **Feniou, Claude, 5 rue du Général Guillaumat, F-33600 Pessac (FR)**
Inventeur: **Grignon, Micheline, 17 rue Raymond Boivin, F-33600 Pessac (FR)**
Inventeur: **Lacourt, Brigitte Résidence la Clairière, Bâtiment Les Morilles 39 rue Louis Vouquet, F-69009 Lyon (FR)**
Inventeur: **Pontagnier, Henri, 21 rue Edouard Vaillant, F-33600 Pessac (FR)**
Inventeur: **Rezzonico, Bernadette Résidence Pierre Curie, Bat F, Appt 198 33 rue Pierre Curie, F-33140 Villenave D'Ornon (FR)**

(74) Mandataire: **Tajan, Marie-Thérèse, LABORATOIRES SARGET Avenue du Président JF Kennedy, F-33701 Mérignac (FR)**

(54) Nouvelle méthode de préparation du D-ribose.

(57) La présente invention concerne une nouvelle méthode de préparation du D-ribose.

Cette méthode de préparation est caractérisée en ce que l'on prépare dans une première étape un dérivé di-0,0-substitué -1,5 du D-xylofurannose, en ce que l'on substitue les hydroxyles situés en 2,3 par un groupement appartenant de préférence au groupe formé par les substituants mésyle $(SO_2CH_3)$ et S-méthyldithiocarbonate $C(=S)SCH_3$, en ce que l'on prépare le dérivé insaturé -2,3, puis enfin en ce que l'on obtient le D-ribose par cis-hydroxylation de l'éthylénique, puis hydrolyse acide du dérivé di-O-alkylé en 1,5.

Le D-ribose ainsi obtenu est utile dans la préparation d'un certain nombre de médicaments.

EP 0 215 722 A1

## Nouvelle méthode de préparation du D-ribose

La présente invention concerne une nouvelle méthode de préparation du D-ribose.

Le D-ribose est un monosaccharide présent à l'état naturel dans les acides ribonucléiques et utile dans la préparation d'un certain nombre de médicaments tels que la riboflavine ou vitamine $B_2$, l'adénosine, l'AMP, la ribavirine et de façon générale de nombreux virostatiques et anticancéreux.

Le D-ribose est fabriqué de façon générale à partir des acides ribonucléiques de microorganismes. On peut également le synthétiser (référence Index Merck, 10°ed., 1983, 8106) à partir du D-érythrose, de l'acide l-glutamique, de l'acide D-ribonique. Toutefois aucune de ces méthodes n'est susceptible d'application industrielle compte tenu de la difficulté des schémas synthétiques proposés et (ou) du prix des matières premières nécessaires.

Nous venons maintenant de découvrir qu'il était possible de synthétiser le D-ribose à partir du D-xylose, monosaccharide largement répandu dans le règne végétal sous forme de xylanes, par une méthode caractérisée en ce que l'on prépare dans une première étape un dérivé di-0,0-substitué -1,5 du D-xylofurannose, de préférence le 0-trityl-5 0-méthyl-1 D-xylofurannoside, en ce que l'on substitue les hydroxyles situés en 2,3 par un groupement appartenant de préférence au groupe formé par les substituants mésyle ($SO_2CH_3$) et S-méthyldithiocarbonate $C(=S)SCH_3$, en ce que l'on prépare le dérivé insaturé -2,3, puis enfin en ce que l'on obtient le D-ribose par cis-hydroxylation de l'éthylénique, puis hydrolyse acide du dérivé di-0-alkylé en 1,5. Ces réactions peuvent être réalisées sur les mélanges d'isomères alpha + béta ou sur l'un ou l'autre des anomères purifiés.

La présente invention va être décrite de façon plus précise dans les exemples suivants sans toutefois que ceux-ci ne limitent sa portée.

## Exemple 1

### Synthèse du O-méthyl-1 D-xylofurannoside

A une suspension de 11 g de D-xylose pulvérisé dans 200 ml de méthanol anhydre, on ajoute sous agitation 50 ml de méthanol contenant 1 g d'acide chlorhydrique gazeux et on poursuit l'agitation pendant 5 heures (la solution est limpide au bout de 2 heures). La solution est neutralisée avec 2,8 g de triéthylamine en agitant une demi-heure et les solvants sont éliminés par évaporation sous pression réduite. Le D-xylose résiduel, en très petite quantité, peut être éliminé par filtration sur colonne de silice (éluant chloroforme-éthanol 100, 8,5-1,5, V/V ; Rf du O-méthyl-1 D-xylofurannoside en chromatographie sur couches minces dans ce système de solvant 0,45). Le produit est utilisé tel quel sans autre purification pour la suite de la réaction. Rendement 95%.

### Synthèse du O-trityl-5 O-méthyl-1 D-xylofurannoside

A une solution de 10 g du composé préalablement préparé dans 40 ml de pyridine anhydre, sont ajoutés par petites portions 17 g de chlorure de trityle. Le milieu est refroidi à 0°C. L'introduction du chlorure de trityle s'accompagne d'une coloration brune du milieu et il y a formation, au bout d'une demi-heure d'agitation, d'un précipité blanc. On laisse la solution revenir à température ambiante et on l'abandonne, sous atmosphère inerte, 48 heures. Le chlorhydrate formé est filtré sur Célite ; la pyridine est éliminée par évaporation sous pression réduite. La phase organique est alors coévaporée deux fois avec du toluène. Le O-trityl-5 O-méthyl-1 D-xylofurannoside est purifié par chromatographie sur colonne de silice (éluant hexane-éthanol, 9-1, V/V ; Rf en chromatographie sur couches minces dans ce système de solvant = 0,20). Il est ainsi obtenu avec un rendement de 85% sous forme d'une mousse blanche.

Synthèse du O-trityl-5 di-O,O-mésyl-2,3 O-méthyl-1 D-xylofurannoside

A une solution de 3 g du composé préalablement préparé dans 15 ml de pyridine anhydre, on ajoute, goutte à goutte, 2,5 ml de chlorure de méthanesulfonyle. Pendant toute la durée de l'addition, le milieu est maintenu à 0°C. On le laisse ensuite revenir à la température ambiante sous atmosphère inerte. Au bout de 15 minutes, le chlorhydrate commence à précipiter. Après un repos de 20 heures, la solution a pris une coloration noire. Le mélange réactionnel est versé dans 40 ml d'un mélange eau-glace et extrait par deux fois 35 ml de chloroforme. Les phases organiques sont regroupées, séchées et évaporées sous pression réduite, puis coévaporées avec deux fois 50 ml de toluène. On obtient le produit attendu sous forme d'une mousse orange ; rendement 91%. Ce produit est purifié par chromatographie sur colonne de silice ( éluant hexane-éthanol, 95-5, V/V). Il est possible d'isoler chacun des anomères ; l'anomère béta est insoluble dans le cyclohexane à chaud et précipite ; l'anomère alpha est obtenu par évaporation des eaux-mères.

Synthèse du O-trityl-5 O-méthyl-1 didésoxy-2,3 D-xylo-penténo-2 furannoside

Dans un ballon, on place 1 g du couple Zn/Cu et 2,5 g d'iodure de potassium en solution dans 10 ml de DMF anhydre. Le montage est balayé par un courant d'argon. A cette suspension et sous agitation, on ajoute goutte à goutte 1,7 g du produit précédemment préparé sous forme d'un mélange 50-50 d'anomères alpha-béta. On chauffe au reflux pendant 4 heures. La solution chaude est versée dans un mélange 50/50 de glace-eau (40 ml) et 30 ml de chloroforme sont ajoutés. On agite pendant 15 mn. Le mélange est filtré sur Célite pour éliminer les sels métalliques et le "gateau" de Célite est lavé deux fois par un mélange 50/50 eau-chloroforme. Les phases chloroformiques sont regroupées, lavées successivement avec une solution saturée de thiosulfate de sodium puis à l'eau, séchées, évaporées sous pression réduite. Presque tout le DMF est éliminé dans la phase aqueuse mais il est nécessaire d'éliminer à la pompe à vide les traces présentes dans la phase organique. Par chromatographie sur colonne de silice (éluant hexane-éthanol 95/5 V/V) on recueille le produit attendu avec un rendement de 70% sous forme d'une huile légèrement jaune.

Le produit obtenu est un mélange d'anomères comportant 80 % de forme béta et 20 % de forme alpha ; on donne ci-après les caractéristiques des spectres de RMN du proton dans $CDCl_3$, avec le tétraméthylsilane comme étalon interne, pour chacun des deux anomères : la première valeur de déplacement chimique correspond à l'anomère béta et la deuxième valeur, entre parenthèses, à l'anomère alpha ; 3,07-3,3 ppm (3,2-3,3 ppm), 2 multiplets, $CH_2$ ; 3,42 ppm (3,38 ppm), 1 singulet, $OCH_3$ ; 4,86 ppm (4,86 ppm), 1 multiplet, $J_{3-4} = 2$ Hz, $J_{2-4} = 1,25$ Hz, C4-H ; 5,74 ppm (5,74 ppm), 1 doublet, $J_{1-2} = 1,25$ Hz, C1-H ; 5,8 ppm (5,84 ppm), 1 multiplet, $J_{1-3} = 1$ Hz, C3-H ; 6,1 ppm (6,24 ppm), 1 doublet, $J_{2-3} = 6$ Hz, C2-H ; 7,16-7,52 ppm (7,16-7,52 ppm), 2 multiplets, $(C_6H_5)_3C$.

Synthèse du O-trityl-5 O-méthyl-1 béta-D-ribofurannoside

A une solution dans 12 ml d'acétone et 6 ml d'eau, refroidie par un bain de glace-sel à -10°C, de 1,15 g de l'anomère béta de l'éthylénique préalablement obtenu, on ajoute goutte à goutte 0,7 g de permanganate de potassium, préalablement pulvérisé et dissous dans un mélange acétone-eau ( 12-4 ml). La température est maintenue constante pendant la durée de l'addition, environ 2 heures. On laisse ensuite le mélange réactionnel revenir à température ambiante. On observe un changement de coloration du rose au marron. Après avoir été maintenu pendant la nuit sous vive agitation le mélange réactionnel est filtré. Les sels de manganèse recueillis sont lavés au dichlorométhane et le filtrat est extrait au dichlorométhane. Les phases organiques sont regoupées, lavées à l'eau et séchées. Le solvant est éliminé par évaporation sous pression réduite. Le produit brut ainsi obtenu avec un rendement de 74% est légèrement jaune. Sa purification est effectuée sur colonne de silice (éluant chloroforme/éthanol 9/1 V/V ; Rf en chromatographie sur couches minces dans ce système de solvant = 0,53). On isole une fraction légèrement jaune, correspondant au beta-riboside, dont le spectre de RMN est identique à celui d'un échantillon préparé à partir de D-ribose selon la double séquence : blocage au méthanol et blocage par le chlorure de triphénylméthane ; rendement 52%.

Le spectre de RMN du proton à 200 MHz a été déterminé dans CDCl$_3$ avec le tétraméthylsilane comme étalon interne : 3,2 ppm, doublet, $J_{4,5}$ = 5,30 Hz, CH$_2$ ; 3,25 ppm, singulet, OCH$_3$ ; 3,94 ppm, doublet, $J_{2,3}$ = 4,75 Hz, C2-H ; 4,01 ppm, multiplet, C4-H ; 4,17 ppm, doublet dédoublé, $J_{3,4}$ = 7,15 Hz, C3-H ; 4,79 ppm, singulet, C1-H ; 7,15 - 7,45 ppm, 2 multiplets, (C$_6$H$_5$)$_3$C ; sur le spectre en onde continue à 60 MHz (dans CDCl$_3$ avec le tétraméthylsilane comme étalon interne), on observe à 3 ppm un massif élargi correspondant aux 2 OH.

## Synthèse du D-ribose

Dans un ballon surmonté d'un réfrigérant, 1 g de 0-trityl-5 0-méthyl-1 ribofurannoside est mélangé à 10 g d'une solution aqueuse 1 N de H$_2$SO$_4$. Le mélange est chauffé sous agitation à 100 °C. Le sucre libéré se dissout dans l'eau tandis que le triphénylcarbinol précipite. Au bout d'une heure, on refroidit à température ambiante, puis on filtre. On neutralise avec une solution aqueuse de soude et on évapore à sec. Le résidu est recristallisé dans 15 cm$^3$ d'éthanol absolu ; le sulfate de sodium insoluble formé lors de la neutralisation est éliminé par filtration. Le D-ribose est ainsi isolé sous forme d'huile. Le produit brut ainsi obtenu est purifié par passage sur colonne de silice avec comme éluant le mélange chloroforme - éthanol (80/20 -V/V). Ses caractéristiques physicochimiques sont les suivantes : point de fusion 87-88°C ; pouvoir rotatoire alpha$_D$ = -26 ( c=3 dans l'eau, à 21°C, lecture au bout de 15 minutes) ; chromatographie sur couches minces d'alumine avec comme éluant le mélange 80 % d'isopropanol et 20 % d'eau : Rf = 0,65.

## Exemple 2

## Synthèse du 0-trityl-5 di-0,0-((méthylthio)thiocarbonyl-2,3 0-méthyl-1 D-xylofurannoside

A une solution de 4,06 g de 0-trityl-5 0-méthyl-1 D-xylofurannose, préparé selon l'exemple 1, dans 70 ml de dichlorométhane, sont ajoutés successivement 6,8 g d'un agent de transfert de phase, l'hydrogénosulfate de tétrabutylammonium, 70 ml de soude à 50%, 2 ml de disulfure de carbone, 3,2 g d'iodure de méthyle. Le mélange devient épais et prend une coloration orange. Il est maintenu sous agitation pendant 30 minutes et la réaction est suivie en chromatographie sur couches minces ( éluant hexane-éthanol, 9-1, V/V ). On ajoute 200 g de glace et on extrait par 200 ml d'éther. Après décantation, la phase aqueuse est extraite deux fois par 100 ml d'éther. Les phases organiques sont ensuite lavées à l'eau, séchées et évaporées sous pression réduite. Le produit brut (rendement 95%) a l'aspect d'une huile. On le purifie par chromatographie sur gel de silice ( éluant hexane-éthanol, 9-1, V/V ). Rendement 94%. Les anomères peuvent être séparés par recristallisation ; l'anomère alpha est obtenu dans le mélange cyclohexane-éthanol/80-20 ; l'anomère béta est récupéré dans les eaux-mères.

Synthèse du O-trityl-5 O-méthyl-1 didésoxy-2,3 D-xylo-penténo-2 furannoside

Dans un ballon, on chauffe au reflux une solution de 3,36 g de $Bu_3SnH$ dans 30 ml de toluène. Sous agitation on ajoute goutte à goutte une solution de 0,85 g du thioester précédemment préparé, dans 30 ml de toluène. Le montage est balayé par un courant d'argon. La réaction est suivie par chromatographie sur couches minces. Le reflux est maintenu pendant 24 heures, temps au bout duquel on note la disparition de la coloration jaune du départ. Le toluène est éliminé par évaporation. On verse le résidu dans 35 ml d'éther et on agite vigoureusement avec une solution saturée de fluorure de potassium jusqu'à la formation d'un précipité blanc. On répète deux fois ces lavages par KF. Les phases organiques sont lavées à l'eau, puis séchées et évaporées sous pression réduite. Après purification par chromatographie sur colonne de silice ( éluant hexane-éther où l'éther est utilisé en pourcentage croissant jusqu'à un mélange final 7-3 V/V ), on obtient une huile jaunâtre ; rendement 50 % si l'on part d'un mélange d'anomères alpha + béta 50/50, 80 % si l'on part de la forme béta pure et 23 % si l'on part de la forme alpha pure.
Le D-ribose est préparé à partir du produit ainsi obtenu selon la méthode décrite dans l'exemple 1.

## REVENDICATIONS

1) Nouvelle méthode de synthèse du D-ribose caractérisée en ce que l'on prépare dans une première étape un dérivé di-0,0-substitué -1,5 du D-xylofurannose, de préférence le 0-trityl-5 0-méthyl-1 D-xylofurannoside, en ce que l'on substitue les hydroxyles situés en 2,3 par un groupement appartenant de préférence au groupe formé par les substituants mésyle $(SO_2CH_3)$ et S-méthyldithiocarbonate $C(=S)SCH_3$, en ce que l'on prépare le didésoxy-2,3-D-xylo-penténo-2 furannoside di-0,0-substitué-1,5, puis enfin en ce que l'on obtient le D-ribose par cis-hydroxylation de cet éthylénique, puis hydrolyse acide du dérivé di-0,0-substitué en 1,5 du ribose.

2) Nouvelle méthode de synthèse du D-ribose selon la revendication 1 caractérisée en ce que l'on prépare dans une première étape un dérivé di-0,0-substitué -1,5 du D-xylofurannose, de préférence le 0-trityl-5 0-méthyl-1 D-xylofurannoside, en ce que l'on substitue les hydroxyles situés en 2,3 par un groupement mésyle $(SO_2CH_3)$, en ce que l'on prépare le didésoxy-2,3-D-xylo-penténo-2 furannoside di-0,0-substitué-1,5, puis enfin en ce que l'on obtient le D-ribose par cis-hydroxylation de cet éthylénique, puis hydrolyse acide du dérivé di-0,0-substitué en 1,5 du ribose.

3) Nouvelle méthode de synthèse du D-ribose selon la revendication 1 caractérisée en ce que l'on prépare dans une première étape un dérivé di-0,0-substitué -1,5 du D-xylofurannose, de préférence le 0-trityl-5 0-méthyl-1 D-xylofurannoside, en ce que l'on substitue les hydroxyles situés en 2,3 par un groupement S-méthyldithiocarbonate $C(=S)SCH_3$, en ce que l'on prépare le didésoxy-2,3-D-xylo-penténo-2 furannoside di-0,0-substitué-1,5, puis enfin en ce que l'on obtient le D-ribose par cis-hydroxylation de cet éthylénique, puis hydrolyse acide du dérivé di-0,0-substitué en 1,5 du ribose.

4) Nouvelle méthode de synthèse du D-ribose selon les revendications 1 à 3 caractérisée en ce que l'on isole l'isomère béta du didésoxy-2,3-D-xylo-penténo-2 furannoside di-0,0-substitué-1,5, avant de procéder à la cis-hydroxylation de cet éthylénique, puis à l'hydrolyse acide du dérivé di-0,0-substitué en 1,5 du ribose.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | CH-A- 425 749 (SANKYO) <br> * Pages 1-3 * <br><br> --- | 1 | C 07 H 3/00 <br> C 07 D 307/00 |
| A | TETRAHEDRON, vol. 30, 1974, pages 3547-3552, Pergamon Press, GB; M. TANIGUCHI et al.: "Stereochemical studies - XXX Stereoselective synthesis of D-ribose form L-glutamic acid" <br> * Pages 3547-3549 * <br><br> --- | 1 | |
| A | CHEMICAL ABSTRACTS, vol. 82, no. 15, 14 avril 1975, page 469, résumé no. 98337f, Columbus, Ohio, US; & JP-A-74 20 172 (TANIGUCHI et al.) 23-05-1974 <br> * Résumé * <br><br> ----- | 1 | |

DOMAINES TECHNIQUES RECHERCHES (Int. Cl 4)

C 07 H
C 07 D

Le présent rapport de recherche a ete etabli pour toutes les revendications

| Lieu de la recherche | Date d'achevement de la recherche | Examinateur |
|---|---|---|
| LA HAYE | 07-10-1986 | VERHULST W. |